Europäisches Patentamt

(19) European Patent Office

Office européen des brevets

(11) EP 0 757 017 B1

(12) EUROPEAN PATENT SPECIFICATION

(45) Date of publication and mention
of the grant of the patent:
20.10.1999 Bulletin 1999/42

(51) Int. Cl.$^6$: C02F 3/12, C02F 3/30,
G01N 33/18

(21) Application number: 96111802.3

(22) Date of filing: 23.07.1996

(54) Method to monitor in liquids the concentration of substances which are degraded by acidifying or alkalizing microorganisms

Verfahren zur Überwachung der Konzentration von Substanzen, die von ansäuernden oder alkanlinisierenden Mikroorganismen in Flüssigkeiten abgebaut werden

Procédé pour surveiller dans des liquides la concentration de substances dégradées par des microorganismes acidifiants ou alcalinisants

(84) Designated Contracting States:
AT BE CH DE DK ES FI FR GB GR IE IT LI LU MC NL PT SE

(30) Priority: 01.08.1995 IT MI951686

(43) Date of publication of application:
05.02.1997 Bulletin 1997/06

(73) Proprietors:
• Rozzi, Alberto
20144 Milano (MI) (IT)
• Massone, Alessandro
20138 Milano (IT)
• Verstraete, Willy
9032 Gent (BE)

(72) Inventors:
• Rozzi, Alberto
20144 Milano (MI) (IT)
• Massone, Alessandro
20138 Milano (IT)
• Verstraete, Willy
9032 Gent (BE)

(74) Representative:
Minoja, Fabrizio, Dr. et al
Bianchetti Bracco Minoja S.r.l.
Via Rossini, 8
20122 Milano (IT)

(56) References cited:
EP-A- 0 049 887          EP-A- 0 531 955
WO-A-90/06900          WO-A-92/01223
DE-A- 3 932 640          US-A- 4 053 743
US-A- 5 403 488

• BOVENDEUR J ET AL: "FIXED-BIOFILM REACTORS IN AQUACULTURAL WATER RECYCLE SYSTEMS: EFFECT OF ORGANIC MATTER ELIMINATION ON NITRIFICATION KINETICS" WATER RESEARCH, vol. 24, no. 2, 1 February 1990, MARSH BARTON, EXETER, DEVON, GB, pages 207-213, XP000169002
• PATENT ABSTRACTS OF JAPAN vol. 10, no. 282 (P-500) < 2338> 25 September 1986 & JP-A-61 100 659 19 May 1986

Remarks:
The file contains technical information submitted after the application was filed and not included in this specification

## Description

The present invention relates to a method to monitor the concentration of those polluting substances, found in liquids such as drinking waters, domestic and industrial wastewaters, which are degraded by acidifying or alkalizing microorganisms. The present invention is mainly related to the control of the concentration of nitrogen, under the ammonium and nitrate forms, and carbohydrates, in the form of organic acids.

## Background of the invention

International and national legislation is becoming more and more strict both for wastewater discharge quality standards and for drinking water requirements. In particular, nutrients quality standards (nitrogen and phosphorous) are more and more severe and the problem of nitrogen control, both in terms of ammonium and nitrates, affects many water treatment plants.

Several processes exploit microbial cultures to degrade complex substances to simpler ones down to final concentrations which are considered acceptable in effluents.

In order to control the quality effluent from a water treatment plant, current process control methods are based on the measurement of a chemical species important to the treatment process.

The successful operation of a water treatment plant requires monitoring and controlling several parameters, a complex and sophisticated instrumentation and qualified personnel.

The present invention exploits microorganisms which are able to transform or degrade substances, especially pollutants and produce protons or hydroxyl ions at the same time.

The instrumentation currently available to monitor. on-line ammonium and nitrate concentrations at low concentrations (to 1 ng/ml) is not very accurate, is expensive and complex, thus requiring qualified personnel. Therefore a simple, accurate and cheap method for such measurements would be easily accepted by the market.

EP 0531955 discloses a process and an apparatus for monitoring the nitrification activity in a sewage tank or flow. The process is performed in a flow cell, by introducing a sample at a predetermined pH, which is corrected by an alkaline substance. The measure of the alkaline substance is related to the nitrification activity.

JP 61-100659 discloses an apparatus for biological measurement of concentration of ammonia, coming from nitrification activity, in an activated sludge. Alkali is injected in a liquid phase so as to keep pH constant, while making the concentration of carbon dioxide in a gaseous phase constant so as to prevent the variation in the concentration of the bicarbonate ion in the liquid of an aeration tank.

## Summary of the invention

The present invention relates to a simple and innovative method to monitor in liquids the concentration of substances or substrates which are degraded by acidifying or alkalizing microorganisms, as described in more detail in the following pages also by means of examples and figures wherein:

Figure 1a shows a basic scheme of the apparatus utilized in the method of the present invention.
Figure 1b shows the automatized version of such apparatus.
Figure 2 shows a diagram of a measurement for a chemical species of interest in an embodiment of the invention with acidifying microorganisms.

According to the present invention, the method can be applied to all kind of acidifying and/or alkalizing microorganisms, which will be defined in the following as "biomass", converting a chemical species defined also as substrate.

The main advantage of the method described in the present invention is the use of a simple system, of low cost which can be serviced even by non-specialized staff.

## Detailed description of the invention

The method according to the present invention includes the following steps:

a) sampling of a known volume of fluid containing both the substrate to be measured and the biomass;

b) introducing said sample in a batch vessel (reactor) at a pH value, said pH value being higher or lower than the equilibrium pH of the sample system for acidifying and alkalizing reactions respectively said equilibrium pH being the value which is reached by the system at the given experimental conditions when the substrate is completely depleted, while a gas selected from oxygen, nitrogen, or air mixed with carbon dioxide or pure air is sparged through the sample;

c) adding a titrating agent to reach the equilibrium pH in the presence of the sparged gas;

d) adding a titrating agent to neutralize the chemical species produced by the degradation or the conversion reaction and keeping the pH set point; in the form of a simple base or acid for pH regulation and control at such a value of equilibrium or alternatively a base or acid solution, which includes one of the chemical species consumed by the degradative reaction in order to control the pH and keep the in-reactor concentration of that substrate constant at the same time;

e) determining the concentration of the substrate as a function of the volume of titrant consumed.

The biomass is either already present in the liquid to be analyzed or can be added to the sample, in the reactor. Biomass can be used both as suspended solids (activated sludge), or as a biofilm attached to a carrier system.

The equilibrium pH is the value which is reached by the system at the given experimental conditions (temperature, composition of the sparged gas) when the substrate is completely depleted. Its value can be modified by varying the $CO_2$ partial pressure of the sparging gas.

The method according to the present invention is described here in a first embodiment which refers to the case of acidifying microorganisms. The titrating solution is a base. In case the microorganism is an alkalizing one, the titrating solution is an acid. In a different embodiment of the present invention, the microorganisms in normal conditions are neither acidifiers nor alkalinizers but they become such when they are given a substrate which produces acidity or alkalinity as a degradation product (e.g. sodium acetate converted to sodium bicarbonate by normal heterotrophs "induced" to behave as alkalinisers).

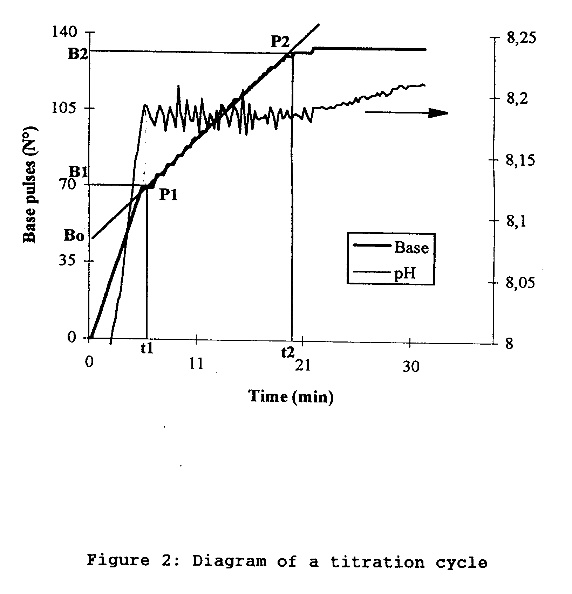

In Fig. 2 a diagram of a titration cycle is reported.

A fixed volume of liquid is first sampled. As described before, the biomass can be either already present in the sample or added as suspended solids or as biofilm fixed to a carrier.

This system, made of the solution containing the substrate and the biomass, is transferred to the reactor and its pH is normally lower than the equilibrium pH of the solution (pH set-point).

The sample therefore contains both the compound (substrate) to be measured and the microorganisms (the active biomass) which degrades it.

At the beginning, the system has to reach the equilibrium pH, preferably in the shortest possible time, and during this phase the pH controller adds the titrating solution at the maximal flow, as indicated in Figure 2 (curve section from 0 to P1, with steepest slope). During this step, the base is mainly added to increase the pH. The equilibrium pH can be reached in the shortest time by the use of additional ad hoc procedures on the titrator, which can be easily determined by the person skilled in the art.

After having reached the optimum pH, the titrating agent is consumed essentially to neutralize the acidity produced by the microorganisms which degrade the substrate and release protons. Thus, as the in-solution pH tends to decrease, this effect is detected by the pH probe which controls the titrating system and adds an amount of alkalinity (given as equivalents) equal to the protons produced (curve section from P1 to P2).

When the substrate is completely consumed, and this conditions is easily detected by the slope of the titrant addition which decreases to zero, the measurement cycle is finished.

To determine the initial concentration of the substrate, the slope (addition velocity) of the titrant curve from P1 to P2 (Figure 2) is used to draw an extrapolating straight line which passes through P1 and intersects the y-axis at point Bo. This point gives the value of the titrating equivalents which are added to reach the set-point pH only. The difference between the point B2, which is the total amount of added base corresponding to point P2, and Bo gives the amount of base equivalents consumed by the microorganisms to produce the protons corresponding to the removed substrate.

Considering the reaction stoichiometry of degradation process ($\alpha$ moles of substrate = 1 eq acid produced = 1 equivalent of titrating base) and the volume V of the sample, it is possible to determine the initial concentration of the substrate: $C = \alpha^*(B2-Bo)/V$ .

The instrument can also be used to measure the concentration of a substrate which produces alkalinity when it is degraded. In this case an acid is utilized as a titrating agent but the principle of operation is the same. In the case that the substrate is nitrate, excess organic carbon is added to the reactor to carry out the determinations.

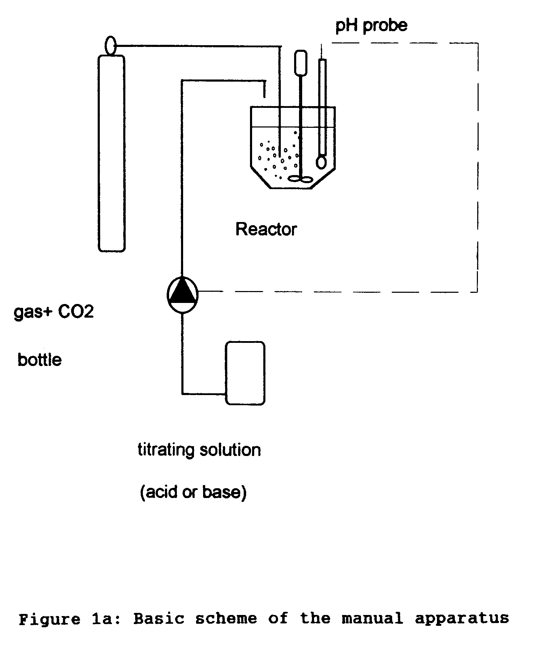

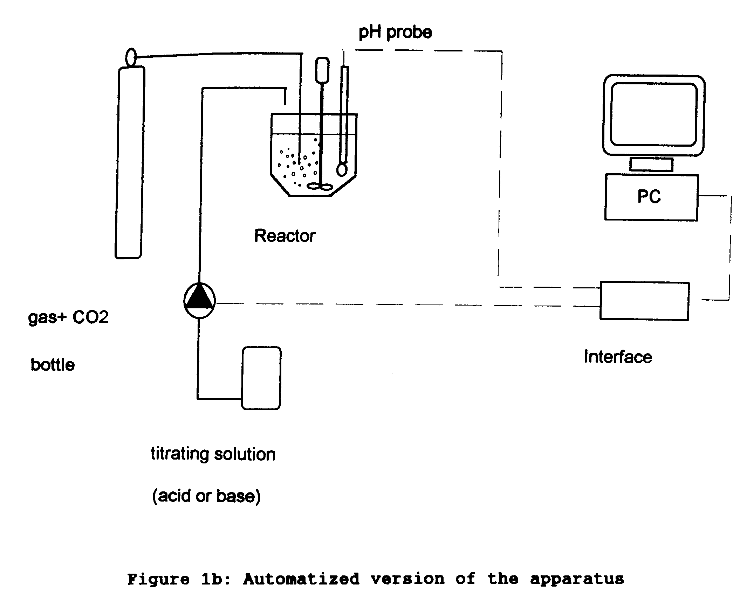

The method described in the present invention makes use of an instrument which is composed of a thermostated vessel with constant volume (about 1 litre), an automatic titrating system controlled by a pH probe, a mixing system, a system for gas storage and sparging, such as a gas bottle and/or ad hoc mixing and dosing systems. Preferably the gas is bubbled in the reaction vessel. The pH probe can be directly submerged in the reactor or fitted in an external cell connected by a recirculation loop to the vessel (design case not indicated in Figure 1). The reactor contents is mixed by a mechanical mixer, with a blade mixer or magnetic mixer or by an external recycle pump. Gas input can be through a sparging porous stone, located within the vessel or in the loop of the recirculation pump.

In a different set-up, the same basic apparatus is automated making use of a suitable I/O interface and a computer, such as a PC that controls the flows in and out of the reactor, the titrating system and acquires and elaborates data by means of a D/A and A/D card.

In figures 1a and 1b two different embodiments of the instrument are shown.

A more simplified (pH controller) apparatus is known and utilized to determine the activity of the acidifying (nitrifyers) microorganisms by means of a slope detection of the titrating agent consumption versus time.

The apparatus used in the method of the present invention allows to determine the activity of the biomass at constant substrate concentrations (steady state conditions) by adding a titrating solution which contains both the neutralizing agent (acid for an alkalizing reaction, base for acidifying reaction) and the consumed substrate which is subsequently reintegrated at the initial concentration.

There are several cases where the acidifying or alkalizing reactions also affect the production or consumption of carbon dioxide, which in turn influence the pH according to the inorganic carbon equilibria. In these cases it is preferable to sparge within the solution sample a gas which contains an appreciable fraction of carbon dioxide, in order to keep the concentration of carbon dioxide in the liquid constant and independent of the biological activity. This procedure makes possible to obtain more accurate determinations.

The apparatus can operate in aerobic, anoxic or anaerobic conditions, depending on the microorganism and chemical species to be determined.

Among the various compounds which can be analyzed with the method described in the present invention are: ammonium, nitrates, polysaccharides and monosaccharides converted to the volatile acids, organics which can be denitrified, phenol, and organic acids such as acetic, citric, lactic acid.

Currently available pHmeters, which can be fitted on the apparatus, allow to determine with acceptable accuracy down to quite low concentrations, of the order of 1 mg/l, ammonium and nitrates. These compounds are difficult to measure at such low concentrations by on-line instrumentation currently available on the market. Moreover the cost of such instruments is quite high, much higher than the one expected for the present apparatus, and require specialized staff for servicing while the analyzer hereby presented is easy to be used even by non-specialized operators.

The apparatus described above, based on the method claimed by the present invention, can be applied to:

- Monitoring and control of industrial and domestic wastewater treatment plants for nitrogen removal (ammonium and nitrate removal).
- Monitoring of readily biodegradable COD in industrial and domestic wastewater treatment plants.
- Monitoring and control of anaerobic digesters by VFA determination.
- Drinking water treatment (nitrates removal).
- Control of fermentation processes in biotechnological and food industries.
- Monitoring sterility in pharmaceutical and food industries.
- Toxicity and inhibition tests on specific and mixed microbial cultures.

## Nitrification of the industrial and civil wastewaters

Nitrification is an aerobic process which requires large amounts of oxygen for ammonium oxidation. Possible nitrogen overloads can be controlled by raising dissolved oxygen concentration in the aeration basins, which increases the nitrification rate. Monitoring on-line influent ammonium concentration on the plant allows a more efficient dosage of the oxygen compared to the currently utilized procedures. Another important advantage of the proposed apparatus is that it allows to detect on-line potentially toxic or inhibiting compounds in the plant influent. The method according to the present invention, when applied to ammonium analysis, allows also to determine the nitrifying activity and thus to monitor and control the process by suitable actions.

According to the present invention, a procedure to control nitrification processes for domestic and industrial wastewaters, comprises the following steps:

a) monitoring of ammonium species using the method described above, steps a) to e) on pages 3-4, using acidifying microorganisms, preferably reaching equilibrium pH in the shortest time possible;

b) monitoring of influent TKN variations using the method described above, steps a) to e) on pages 3-4, using acidifying microorganisms, preferably reaching equilibrium pH in the shortest time possible;

c) monitoring of the nitrate species utilizing the method described above, steps a) to e) on pages 3-4, using alkalizing microorganisms, preferably reaching equilibrium pH in the shortest time possible.

## Denitrification of civil and industrial wastewaters

In order to remove nitrogen from wastewater, nitrification of ammonium to nitrate requires, as a subsequent step, biological denitrification. In many wastewaters, both civil and industrial, the carbon/nitrogen ratio is too low for denitrification purposes. It is thus necessary to add an external carbon source in excess of nitrate reduction requirements.

The dosage of such carbon source obviously depends on nitrate concentration. On-line monitoring of such parameter allows a dosage of the carbon source tuned to nitrogen load and consequently appreciable savings and improved reliability of the process to meet the required discharge standards.

To obtain a complete control of the process it is useful to measure (by the previous application of the biosensor) influent ammonium concentration which is normally proportional to the total nitrogen concentration (TKN). This procedure allows to estimate in advance the total concentration of nitrogen which could be oxidized to nitrate.

According to the present invention, a procedure to control denitrification processes for domestic and industrial wastewaters, comprises monitoring of the nitrate species utilizing the method described above, with alkalizing microorganisms, preferably reaching equilibrium pH in the shortest time possible.

## BOD concentration monitoring in industrial wastewaters

The method and the instrument of the present invention, in a version using alkalizing denitrifying microorganisms, allows to monitor the concentration of organic substrate in wastewaters mainly containing BOD and rapidly biodegradable COD (rbCOD, in this case oxidized by a denitrification reaction). In order to obtain accurate determinations, the composition of the wastewater (as classes of substances) must be relatively constant while variations of flow and total concentration are irrelevant. Most wastewaters from the agro-industries have these characteristics.

In the reaction vessel, which contains biomass plus nitrates in excess, a fixed amount of wastewater to be monitored is added. Alkalinity produced by nitrates reduction is neutralized by titrating with a nitric acid solution and thus the amount of nitrates in the reactor volume is kept constant. The consumption of nitrates, which have been reduced by the biodegraded COD from the wastewater sample, is measured. Based on this amount of reduced nitrates, it is then possible to estimate the amount of rapidly biodegradable COD present in the sample and thus to control the process.

## Monitoring of VFA for anaerobic digesters control

Efficient process control of anaerobic digesters can be obtained by monitoring the in-reactor concentration of volatile fatty acids (VFA), mainly acetic, propionic and butyric acids.

The instrument of the invention, in the denitrification version for readily biodegradable COD monitoring, can be used to determine in anaerobic digesters the concentration of VFA, which are the most easily biodegradable substrates in this type of reactor.

The principle of operation is the same as in the previous case which allows to estimate total VFA concentration. Moreover, taking into account that denitrification velocities of propionic acid is appreciably different from those related to acetic and butyric acids, calibration curves of denitrification activity carried out with individual additions of acids (Acetic and Butyric) would allow to obtain an approximated evaluation of the ratio propionic acid/TVA. These two parameters are considered among the most important for effective anaerobic process control.

According to the present invention, a procedure to monitor the nitrification velocity and ammonia concentration in Sequency Batch Reactors (SBR) in order to define exactly the time length of the nitrification phase as a function of the final concentration of ammonia required in the effluent is performed with the claimed method, using acidifying microorganisms.

According to the present invention, a procedure to monitor denitrification velocity and nitrate concentration in SBR reactors in order to define exactly the time length of the denitrification phase as a function of the final concentration of nitrate required in the effluent is performed with the claimed method, using an alkalizing microorganism.

## Nitrates removal from drinking waters

The availability of drinking water, which is normally drawn from surface and groundwater sources, is becoming more and more difficult due to the enormous variety of organic and inorganic pollutants which can contaminate the water supplies. Among the most diffused inorganic pollutants are nitrates, leaching from fields after addition of nitrogen fertilizers.

Several processes are available for nitrate removal. At present biological denitrification is considered the one which minimizes pollution of concentrated streams which are produced by other processes such as ion exchange and membrane separations. The optimization of the minimum dosage of external carbon, which is usually a problem, is completely solved by the method hereby described.

The apparatus, in the version for alkalizing microorganisms, allows an accurate control of nitrates concentration in the effluent of the denitrifying reactor by an appropriate dosing of the carbon source flow, with appreciable savings in reagents consumption.

## Control of fermentation processes in food and biotechnological industries

Fermentation processes, especially those processes based on the consumption of carbohydrates (such as the lactic fermentation from glucose), include in most cases an acidification step of the culture broth. To control the reaction kinetics, substrate concentration must be monitored. This goal can be obtained by the indirect measurement of fermentation products by means of the method of the present invention.

## Monitoring sterility in pharmaceutical and food industries

As already mentioned, acidification of simple organic substrates such as glucose is a very common reaction for many microbial populations. This also applies to microorganisms which could potentially contaminate sterile environments. In these cases, addition of simple carbohydrates to a sample of the solution which could be contaminated and subsequent titration in the biosensor described in this invention allows to detect non sterile conditions. The method could be applied using other readily biodegradable carbon sources than exoses.

## Inhibition and toxicity tests on specific and mixed microbial cultures

Contrary to the previous applications which mainly refer to on-line monitoring of some chemical and of bacterial populations, the uses proposed in this paragraph refer to laboratory determinations. These applications are most important during preliminary screening studies which must be performed to evaluate the biological treatability of industrial or other toxic wastewaters.
This methodology can be applied to autotrophic and heterotrophic aerobic bacteria as well as to heterotrophic microbial populations which operate in anoxic and anaerobic conditions.

*Aerobic cultures - autotrophs*

Among the aerobic microbial species most sensitive to toxicants are the nitrifyers (autotrophic bacteria). Very rapid and accurate inhibition or toxicity tests can be performed on these microorganisms by keeping excess ammonium in the biosensor reaction vessel and adding increasing amounts of the toxicant to be assayed. The variation of titrant consumption rate, i.e. the variation of the nitrification activity, allows a direct determination of the inhibiting effect.

*Aerobic cultures - heterotrophs*

This methodology can also be applied to bacteria which are not acidifyers or alkalinizers in normal operating conditions but which can be induced to become so by providing them with a suitable substrate such as an organic acid salt. If, e.g., sodium acetate is added to a culture of aerobic heterotrophs, sodium bicarbonate is produced which alters (raises) the pH of the mixed liquor. By adding a titrating acid solution which neutralizes the alkalinity, the consumption of substrate can be determined. Operating in excess substrate concentration and adding increasing amounts of toxicant it is possible to measure the inhibiting effect as indicated above.

*Denitrifyers (anoxic cultures)*

This technique can be equally used to determine inhibiting and toxic effects on mixed cultures of denitrifying microorganisms. In this case excess nitrate and readily biodegradable carbon source are kept in the biosensor reactor vessel.

*Anaerobic cultures*

The method can be used to measure fermentative activity of acidogenic cultures as shown before and to verify the activity of acetoclastic methanogens in mixed cultures where the production of methane by the hydrogenotrophic methanogens could be a high fraction of the total and when it is not possible or practical to measure the production of biogas and its composition.

## Biodegradability studies of specific substrates

In some cases it can be useful to know the rapidly biodegradable fraction related to wastewaters or to concentrated organic wastes. This is particularly important when these wastes are considered as a potential inexpensive carbon source for denitrification of wastewaters with unfavourable C/N ratio.
The proposed biosensor (in the denitrification version) allows to determine not only the readily biodegradable COD in a waste which can be used as a carbon source for denitrification but also amount of nitrate which is reduced and how the denitrification rate decreases as the function of the fraction of substrate which becomes less and less biodegradable.

## Claims

1. A method to monitor in liquids the concentration of substances or substrates which are degraded by acidifying or alkalinizing microorganism, comprising the following steps:

    a) sampling of a known volume of fluid containing both the substrate to be measured and the biomass;
    b) introducing said sample in a batch vessel (reactor) at a pH value, said pH value being higher or lower than the equilibrium pH of the sample system for acidifying and alkalizing reactions respectively, said equilibrium pH being the value which is reached by the system at the given experimental conditions when the substrate is completely depleted, while a gas selected from oxygen, nitrogen, or air mixed with carbon dioxide or pure air is sparged through the sample;
    c) adding a titrating agent to reach the equilibrium pH in the presence of the sparged gas;
    d) adding a titrating agent to neutralize the chemical species produced by the degradation reaction of the substrate and keeping the pH at the equilibrium pH of the solution (pH setpoint);
    e) determining the concentration of the substrate in the sample as a function of the volume of the titrating agent consumed.

2. The method according to claim 1, in which a given volume of suspension (sludge) containing the biomass is added to the sample of fluid containing only the substrate.

3. The method according to claim 1, in which the biomass is already present in the reactor as fixed biofilm attached to a suitable carrier.

4. The method according to claim 1, in which the equilibrium pH related to step c) is controlled by changing the partial pressure of the $CO_2$ in the sparging gas.

5. The method according to claim 1, in which the microorganisms are acidifiers.

6. The method according to claim 1, in which the microorganisms are alkalizers.

7. The method according to claim 1, in which the microorganisms in normal conditions are neither acidifiers nor alkalinizers, but they become such when they are given a substrate which produces acidity or alkalinity as a degradation product.

8. The method according to claim 1, in which the time to reach the equilibrium pH in step c) is minimized.

9. The method according to claims 1-8, in which the chemical species which is determined in step e) is selected from: ammonium, nitrate, phenol, organic acids, carbohydrates.

10. The method according to claims 1-9 whereby an apparatus is used constituted by a thermostated reactor, at constant volume, an automatic titrating system controlled by a pH probe, mixing systems and a gas sparging systems.

11. The method according to claim 10, in which such apparatus comprises also a suitable input/output interface, an electronic controlling computer device plus a data processing system.

12. Procedure to control nitrification processes for domestic and industrial wastewaters comprising the following steps:

    a) Monitoring of an ammonium species using the method of claims 1-5, 8 and of claims 10-11;
    b) Monitoring of influent TKN variations using the method of claims 1-5, 8 and of claims 10-11;
    c) Monitoring of the nitrate species utilizing the method of claims 1-4, 6 and 8 and of claims 10-11.

13. Procedure to control denitrification processes for domestic and industrial wastewaters in which monitoring of the nitrate species utilizing the method of claims 1-4, 6 and 8 and of claims 10-11.

14. Procedure to monitor in wastewaters the concentration of readily biodegradable COD by using the method of claims 1-4, 6 and 8 and of claims 10-11, wherein alkalizing denitrifying microorganisms are used.

15. Procedure to monitor the nitrification velocity and ammonia concentration in SBR reactors in order to define exactly the time length of a nitrification phase as a function of a final concentration of ammonia required in the effluent with the method of claims 1-5, 8 and of claims 10-11.

16. Procedure to monitor denitrification velocity and nitrate concentration in SBR reactors in order to define exactly the time length of the denitrification phase as a function of the final concentration of nitrate required in an effluent using the method of claims 1-4, 6 and 8 and of claims 10-11.

17. Procedure for nitrate removal from potable waters is controlled in which nitrate species control is performed utilizing the method of claims 1-4, 6 and 8 and of claims 10-11.

18. Procedure to monitor and control a fermentation process having as a product an organic or inorganic acid in which the concentration of the acid is actuated with the method of claims 1-5, 8 and of claims 10-11.

19. Procedure to monitor the presence of contaminating microorganisms in sterile waters by adding carbohydrate or other rapidly fermentable organics to a sample of the potentially contaminated waters and determining the presence of the organisms by an appreciable acidification in the reactor, according to the method of claims 1-5, 8 and of claims 10-11.

20. Procedure to determine the inhibiting effects exerted on non-acidifying or alkalizing microorganisms by a toxic chemical by using a substrate which produces acidity or alkalinity when it is biodegraded and allows to measure variations of biological activity in the presence of a toxicant according to the method of claims 1-4, 6 and 8 and of claims 10-11.

**Patentansprüche**

1. Verfahren zur Überwachung der Konzentration von Substanzen oder Substraten, die durch säurebildende oder alkalinisierende Mikroorganismen abgebaut werden, in Flüssigkeiten, umfassend die folgenden Stufen:

    a) Entnahme eines bekannten Volumens einer Flüssigkeit, die sowohl das zu messende Substrat als auch die Biomasse enthält;

b) Zugabe der Probe in ein Chargengefäß (Reaktor) bei einem pH-Wert, wobei der pH-Wert höher oder niedriger ist als der Gleichgewichts-pH des Probensystems für die säurebildenden bzw. alkalinisierenden Reaktionen, wobei der Equilibrium-pH derjenige Wert ist, der von dem System unter gegebenen experimentellen Bedingungen erreicht wird, wenn das Substrat vollständig erschöpft ist, wobei währenddessen ein Gas, ausgewählt aus Sauerstoff, Stickstoff oder mit Kohlendioxid gemischte Luft oder reine Luft, durch die Probe zerstäubt wird;

c) Zugabe eines Titrierungsmittels, um in Gegenwart des Zerstäubungsgases den Gleichgewichts-pH zu erreichen;

d) Zugabe eines Titrierungsmittels, um die chemische Spezies zu neutralisieren, die durch die Abbaureaktion des Substrates erzeugt wird und um den pH beim Gleichgewichts-pH der Lösung zu halten (pH-Sollpunkt);

e) Bestimmung der Konzentration des Substrats in der Probe als Funktion des Volumens verbrauchten Titrationsmittels.

2. Verfahren nach Anspruch 1, bei dem ein gegebenes Suspensionsvolumen (Schlamm), das die Biomasse enthält, der Probe der Flüssigkeit, die lediglich das Substrat enthält, zugegeben wird.

3. Verfahren nach Anspruch 1, bei dem die Biomasse bereits im Reaktor als fixierter Biofilm, der an einen geeigneten Träger gebunden ist, vorhanden ist.

4. Verfahren nach Anspruch 1, bei dem der Equilibrium-pH, der die Stufe c) betrifft, durch Änderung des Partialdrucks des $CO_2$ im Zerstäubungsgas kontrolliert wird.

5. Verfahren nach Anspruch 1, bei dem die Mikroorganismen säurebildende Mikroorganismen sind.

6. Verfahren nach Anspruch 1, bei dem die Mikroorganismen alkalisierende Mikroorganismen sind.

7. Verfahren nach Anspruch 1, bei dem die Mikroorganismen bei normalen Bedingungen weder säurebildende noch alkalisierende Mikroorganismen sind, jedoch säurebildend oder alkalisierend werden, wenn sie ein Substrat erhalten, das Azidität oder Alkalinität als Abbauprodukt bildet.

8. Verfahren nach Anspruch 1, bei dem die Zeit zur Erreichung des Equilibrium-pH bei der Stufe c) minimiert ist.

9. Verfahren nach den Ansprüchen 1 bis 8, bei dem die chemische Spezies, die in der Stufe e) bestimmt wird, ausgewählt ist aus Ammonium, Nitrat, Phenol, organischen Säuren, Kohlenhydraten.

10. Verfahren nach den Ansprüchen 1 bis 9, wobei ein Apparat verwendet wird, der aus einem temperaturgeregelten Reaktor, bei konstantem Volumen, einem durch eine pH-Sonde kontrollierten automatischen Titrationssytem, Mischsystemen und Gaszerstäubungssystemen besteht.

11. Verfahren nach Anspruch 10, bei dem ein derartiger Apparat auch ein geeignetes Eingangs-/Ausgangsinterface (input/output interface), eine elektronisch kontrollierende Computereinrichtung plus ein Datenprozessierungssystem umfaßt.

12. Verfahren zur Kontrolle von Nitrifizierungsverfahren von Haushalts- und Industrieabwässern, umfassend die folgenden Stufen:

a) Überwachung einer Ammoniumspezies unter Verwendung des Verfahrens nach den Ansprüchen 1 bis 5, 8 und den Ansprüchen 10 bis 11;

b) Überwachung der Zufluß-TKN-Variationen unter Verwendung des Verfahrens nach den Ansprüchen 1 bis 5, 8 und den Ansprüchen 10 bis 11;

c) Überwachung der Nitratspezies unter Verwendung des Verfahrens nach den Ansprüchen 1 bis 4, 6 und 8 und den Ansprüchen 10 bis 11.

13. Verfahren zur Kontrolle von Denitrifizierungsverfahren für Haushalts- und Industrieabwässer, bei dem die Nitratspezies von der Verwendung des Verfahrens nach den Ansprüchen 1 bis 4, 6 und 8 und den Ansprüchen 10 bis 11 überwacht wird.

14. Verfahren zur Überwachung der Konzentration schnell abbaubaren CODs in Abwässern unter Verwendung des Verfahrens nach den Ansprüchen 1 bis 4, 6 und 8 und den Ansprüchen 10 bis 11, wobei alkalinisierende denitrifizierende Mikroorganismen verwendet werden.

15. Verfahren zur Überwachung der Nitrifizierungsgeschwindigkeit und der Ammoniakkonzentration in SBR-Reaktoren, um die Zeitdauer einer Nitrifizierungsphase als Funktion einer Endkonzentration benötigten Ammoniaks im ausfließenden Medium mit dem Verfahren nach den Ansprüchen 1 bis 5, 8 und den Ansprüchen 10 bis 11 genau zu definieren.

16. Verfahren zur Überwachung der Denitrifizierungsgeschwindigkeit und der Nitratkonzentration in SBR-Reaktoren, um die Zeitdauer der Denitrifizierungsphase als Funktion der Endkonzentration benötigten Nitrats im ausfließenden Medium unter Verwendung des Verfahrens nach den Ansprüchen 1 bis 4, 6 und 8 und den Ansprüchen 10 und 11 genau zu definieren.

17. Verfahren zur Kontrolle der Nitratentfernung aus Trinkwasser, wobei die Nitratspezieskontrolle unter Verwendung des Verfahrens nach den Ansprüchen 1 bis 4, 6 und 8 und den Ansprüchen 10 bis 11 durchgeführt wird.

18. Verfahren zur Überwachung und Kontrolle eines Fermentationsverfahrens, dessen Produkt eine organische oder anorganische Säure ist, bei dem die Konzentration der Säure mit dem Verfahren nach den Ansprüchen 1 bis 5, 8 und den Ansprüchen 10 bis 11 gesteuert wird.

19. Verfahren zur Überwachung des Vorhandenseins kontaminierender Mikroorganismen in sterilen Wässern durch Zugabe von Kohlenhydraten oder anderer schnell vergärbarer organischer Substanzen zu einer Probe des möglicherweise kontaminierten Wassers und Bestimmung des Vorhandenseins der Organismen mittels einer merklichen Ansäuerung im Reaktor nach dem Verfahren der Ansprüche 1 bis 5, 8 und der Ansprüche 10 bis 11.

20. Verfahren zur Bestimmung der inhibitorischen Wirkungen einer toxischen Chemikalie auf nicht-säurebildende oder alkalisierende Mikroorganismen unter Verwendung eines Substrates, das eine Azidität oder Alkalinität erzeugt, wenn es biologisch abgebaut wird und das es möglich macht, Variationen der biologischen Aktivität in Gegenwart eines Giftstoffes nach dem Verfahren der Ansprüche 1 bis 4, 6 und 8 und den Ansprüchen 10 bis 11 zu messen.

## Revendications

1. Procédé pour contrôler, dans des liquides, la concentration de substances ou substrats qui sont dégradés par des micro-organismes acidifiants ou alcanisants, comprenant les étapes consistant à :

   a) échantillonner un volume connu de fluide contenant à la fois le substrat à mesurer et la biomasse ;
   b) introduire ledit échantillon dans une cuve à fonctionnement discontinu (réacteur) à une valeur de pH, ladite valeur de pH étant supérieure ou inférieure au pH d'équilibre du système constituant l'échantillon respectivement pour des réactions d'acidification et d'alcanisation, ledit pH d'équilibre étant la valeur atteinte par le système dans les conditions expérimentales données lorsque le substrat est totalement épuisé, alors qu'un gaz choisi parmi l'oxygène, l'azote, ou l'air mélangé au dioxyde de carbone ou l'air pur est mis à barboter dans l'échantillon ;

   c) ajouter un agent de titrage pour obtenir le pH d'équilibre en présence du gaz de barbotage ;
   d) ajouter un agent de titrage pour neutraliser l'espèce chimique produite par la réaction de dégradation du substrat et maintenir le pH au pH d'équilibre de la solution (pH de référence) ;
   e) déterminer la concentration du substrat dans l'échantillon en fonction du volume de l'agent de titrage consommé.

2. Procédé selon la revendication 1 dans lequel un volume donné de suspension (bouillie) contenant la biomasse est ajouté à l'échantillon de fluide contenant uniquement le substrat.

3. Procédé selon la revendication 1 dans lequel la biomasse est déjà présente dans le réacteur sous forme de biofilm fixe lié à un support approprié.

4. Procédé selon la revendication 1 dans lequel le pH d'équilibre relatif à l'étape c) est contrôlé en modifiant la pression partielle de $CO_2$ dans le gaz de barbotage.

5. Procédé selon la revendication 1 dans lequel les micro-organismes sont des acidifiants.

6. Procédé selon la revendication 1 dans lequel les micro-organismes sont des alcalinisants.

7. Procédé selon la revendication 1 dans lequel, dans des conditions normales, les micro-organismes ne sont ni des acidifiants ni des alcalinisants, mais le deviennent lorsqu'il reçoivent un substrat qui, en tant que produit de dégradation, génère un caractère acide ou alcalin.

8. Procédé selon la revendication 1 dans lequel le temps nécessaire pour atteindre le pH d'équilibre au cours de l'étape c) est minimisé.

9. Procédé selon l'une quelconque des revendications 1 à 8, dans lequel l'espèce chimique défini à l'étape e) est choisi parmi l'ammonium, un nitrate, un phénol, les acides organiques, les hydrates de carbone.

10. Procédé selon l'une quelconque des revendications 1 à 9, dans lequel est utilisé un appareil qui est constitué par un réacteur thermostaté à un volume constant, un système automatique de titrage contrôlé par une sonde mesurant le pH, des systèmes de mélange et des systèmes de barbotage de gaz.

11. Procédé selon la revendication 10, dans lequel ce dispositif comprend également une interface entrée/sortie appropriée, un dispositif constituant un ordinateur de contrôle électronique, et un système de traitement de données.

12. Procédé pour contrôler des opérations de nitrification destinées à des eaux usées issues d'un usage domestique ou industriel, comprenant les étapes consistant à :

a) surveiller une espèce ammonium en utilisant le procédé selon les revendications 1 à 5, 8, et les revendications 10 et 11 ;
b) surveiller les variations de TKN d'entrée en utilisant le procédé selon les revendications 1 à 5, 8 et les revendications 10 et 11 ;
c) surveiller l'espèce nitrate en utilisant le procédé selon les revendications 1 à 4, 6 et 8 et les revendications 10 et 11.

13. Procédé pour contrôler les opérations de dénitrification destinées à des eaux usées issues d'un usage domestique ou industriel, dans lequel on surveille l'espèce nitrate en utilisant le procédé selon les revendications 1 à 4, 6 et 8 et les revendications 10 et 11.

14. Procédé pour surveiller, dans des eaux usées, la concentration de la DCO facilement biodégradable en utilisant le procédé selon les revendications 1 à 4, 6 et 8 et les revendications 10 et 11, dans lequel des micro-organismes d'acalnisation et de dénitrification sont utilisés.

15. Procédé pour surveiller la vitesse de nitrification et la concentration d'ammoniac dans des réacteurs SBR pour définir de manière exacte la durée de la phase de nitrification en fonction de la concentration finale d'ammoniac requise dans l'effluent en utilisant le procédé selon les revendications 1 à 5, 8 et les revendications 10 et 11.

16. Procédé pour surveiller la vitesse de dénitrification et la concentration en nitrate dans des réacteurs SBR afin de définir de manière exacte la durée de la phase de dénitrification en fonction de la concentration finale de nitrate requise dans un effluent en utilisant le procédé selon les revendications 1 à 4, 6 et 8 et les revendications 10 et 11.

17. Procédé pour contrôler l'elimination du nitrate de l'eau potable dans lequel le contrôle de l'espèce nitrate est réalisé en utilisant le procédé selon les revendications 1 à 4, 6 et 8 et les revendications 10 et 11.

18. Procédé pour surveiller et contrôler un procédé de fermentation dont le produit est un acide organique ou inorganique, dans lequel la concentration de l'acide est commandée par le procédé selon les revendications 1 à 5, 8 et les revendications 10 et 11.

19. Procédé pour surveiller la présence de microorganismes contaminants dans des eaux stériles en ajoutant un hydrate de carbone ou d'autres produits organiques pouvant être fermentés rapidement à un échantillon d'eaux susceptibles d'être contaminées et pour déterminer la présence des organismes par une acidification notable dans le réacteur, selon le procédé objet des revendications 1 à 5, 8 et des revendications 10 et 11.

20. Procédé pour déterminer les effets inhibants exercés sur des micro-organismes ne présentant pas de caractère acidifiant ou alcanisant par un produit chimique toxique, en utilisant un substrat qui génère un caractère acide ou alcalin lorsqu'il est biodégradé et permet de mesurer des variations de l'activité biologique en présence d'un produit toxique selon le procédé des revendications 1 à 4, 6 et 8 et des revendications 10 et 11.

pH probe

Reactor

gas+ CO2

bottle

titrating solution

(acid or base)

Figure 1a: Basic scheme of the manual apparatus

Figure 1b: Automatized version of the apparatus

Figure 2: Diagram of a titration cycle